Europäisches Patentamt

European Patent Office

Office européen des brevets

(11) Veröffentlichungsnummer: **0 203 561 B1**

## (12) EUROPÄISCHE PATENTSCHRIFT

(45) Veröffentlichungstag der Patentschrift: **28.08.91**

(51) Int. Cl.5: **G01N 27/00**

(21) Anmeldenummer: **86107134.8**

(22) Anmeldetag: **26.05.86**

(54) **Betriebsverfahren für einen Sensor für Gasanalyse.**

(30) Priorität: 30.05.85 DE 3519410

(43) Veröffentlichungstag der Anmeldung:
03.12.86 Patentblatt 86/49

(45) Bekanntmachung des Hinweises auf die
Patenterteilung:
28.08.91 Patentblatt 91/35

(84) Benannte Vertragsstaaten:
AT BE CH DE FR GB IT LI NL SE

(56) Entgegenhaltungen:
EP-A- 0 157 237
DE-A- 3 030 448
US-A- 3 961 248
US-A- 4 584 867

(73) Patentinhaber: **Siemens Aktiengesellschaft
Wittelsbacherplatz 2
W-8000 München 2(DE)**

(72) Erfinder: **Müller, Rudolf, Prof. Dr.
Ahornweg 22
W-8135 Söcking(DE)**
Erfinder: **Lange, Eckhard, Dipl.-Ing.
Siedlungsstrasse 1
W-8191 Gelting(DE)**
Erfinder: **Schweizer, Erich, Dipl.-Ing.
Conolly-Strasse 3/F607
W-8000 München 40(DE)**

**Beschreibung**

Die vorliegende Erfindung bezieht sich auf ein Verfahren nach dem Oberbegriff des Patentanspruches 1.

Aus der US-A- 39 99 122 ist ein Gassensor mit einem zur Detektion verwendeten Halbleiterelement und mit einer Selektiveinrichtung bekannt. Das Halbleiterelement ist ein Feldeffekttransistor mit Source, Drain und einem zwischen Source und Drain sich erstreckendem, bis an die Oberfläche des Halbleiterkörpers des Elementes reichenden Kanalbereich. Auf der Oberfläche dieses Halbleiterkörpers befindet sich diesen Kanalbereich überdeckkend als Selektiveinrichtung eine Schicht aus $\beta$-Carotin. Bekanntermaßen ist dieses Carotin ein für Gase sensitiver Stoff und seine Verwendung führt bei dem Halbleiter-Feldeffekttransistor zu Ladungsinfluenzierung im Kanalbereich, zu unterschiedlichem Leitungsverhalten des Feldeffekttransistors und/oder zu geänderter Schwellenspannung. Weitere Gassensoren allgemeiner Art sind aus der US-A- 4 020 830, US-A- 4 332 658 und US-A- 4 499 423 und US-A- 4 457 161 bekannt.

Zur Detektion von Wasserstoff, der auch in wasserstoffhaltiger Verbindung vorliegen kann, sind in Appl. Phys. Letters, Bd. 26 (1975), S. 55 - 57 Gassensoren beschrieben, die im wesentlichen aus einem MOS-Transistor bestehen, dessen Gate-Elektrode aus Palladium besteht. Palladium ist wie z.B. auch Rhodium ein Metall, das katalytische Wirkung für Wasserstoff hat und atomaren Wasserstoff aus molekularen Wasserstoffverbindungen abzuspalten vermag. Der atomare Wasserstoff diffundiert durch das Palladiummetall der Gate-Elektrode hindurch an die zwischen Elektrode und Halbleiteroberfläche befindliche Oxidschicht des Transistors. Der dort absorbierte Wasserstoff bewirkt das Entstehen einer Dipolschicht, durch deren Vorhandensein sich das Maß der Schwellenspannung des Transistors verändert.

Ein wie voranstehend beschriebener Gassensor läßt sich nicht für wasserstofffreie Gase verwenden. Für eine dementsprechende Gasdetektion ist vorzugsweise für CO-Nachweis in "ESSDERC", München, Sept. 1979, in "Int. Vac. Conf.", Cannes, Sept. 1980 und in IEEE Trans. ED 26 (1979), S. 390 - 396, ein MQS-Transistor beschrieben, dessen Gate-Elektrode vorzugsweise wiederum aus Palladium besteht, jedoch diese Palladiumelektrode besitzt eine Vielzahl bis zur Metalloxid-Grenzschicht reichende Löcher.In dem Zusammenhang kommt auch die Verwendung eines NMOS-Transistors in Frage. Solche Transistoren mit perforiertem Palladiumgate haben eine gute Empfindlichkeit für Kohlenmonoxid und stark verminderte "Quer"-Empfindlichkeit gegenüber Wasserstoff. Als Querempfindlichkeit wird hier eine zusätzlich zur eigentlichen gewünschten Empfindlichkeit des Sensors hinzukommende Empfindlichkeit in bezug auf ein anderes Gas bezeichnet. Für bekannte Anordnungen ist das Maß der Änderung der Schwellenspannung in Abhängigkeit von der Gaskonzentration bekannt, wobei eine weitgehend lineare Abhängigkeit zu beobachten ist. Als nachteilig angesehen wird, daß das Ansprechen eines derartigen Gassensors ein dynamischer Prozeß ist, der mit einer gewissen zeitlichen Verzögerung auf die Einwirkung des betreffenden Gases, z.B. des Kohlenmonoxids, einsetzt. In der obengenannten US-A- 4 499 423 ist die Anwendung elektronischer Maßnahmen angegeben, mit denen diese als nachteilig angesehene Zeitabhängigkeit kompensiert wird.

Ergänzend sei erwähnt, daß eine gegebene Querempfindlichkeit eines jeweiligen Sensors durch Zusatzmaßnahmen vermindert werden kann. Zum Beispiel kann bei einem wie zuletzt beschriebenen CO-Sensor die Querempfindlichkeit hinsichtlich Wasserstoffs durch eine aufgebrachte spezielle Schutzschicht um mindestens mehr als eine Größenordnung reduziert werden. Außerdem ist auch zu erwähnen, daß die quantitative Empfindlichkeit und auch die Verzögerungs-Zeitkonstante temperaturabhängig sind.

Bei voranstehend beschriebenen Gassensoren wurde Palladium verwendet. Als wasserstoffdurchlässig sind außerdem auch Rhodium, Platin und Nickel bekannt. Silber besitzt eine ausgeprägte selektive Durchlässigkeit für Sauerstoff.

Es ist auch ein Gassensor (der Firma Figaro) mit gesintertem Zinndioxid für brennbare und für einige toxische Gase bekannt, der auf der Basis einer Widerstandsänderung des leitend gemachten Zinndioxids beruht.

Unter dem Namen "Pellistor" sind Gassensoren bekannt, die nach dem Prinzip der Klorimetrie arbeiten. Ein Pellistor besteht aus zwei Platinwiderstandsdrähten, auf die je eine poröse Keramikpille aufgesintert ist. Auf eine der beiden Keramikpillen ist ein Katalysator aufgebracht. Bei katalytischer Verbrennung des nachzuweisenden Gases ergibt sich für den Platinwiderstandsdraht mit der mit Katalysator beschichteten Keramikpille eine meßbare Widerstandserhöhung, nämlich gegenüber dem zweiten Platinwiderstandsdraht, wobei zur Messung diese beiden Platinwiderstandsdrähte in eine Brückenschaltung eingefügt sind.

Kalorimetrische Effekte im Zusammenhang mit Katalysatoren sind aus dem Stand der Technik bekannt. Es sind dies die Verbrennung von Wasserstoff an einem Platinkatalysator, die Erzeugung von NO aus $NH_3$ mit Platin oder Platin-Rhodium als Katalysator bei 200 bis 250° C und von $NO_2$ aus NO mit einem Katalysator aus $Al_2O_3$-$SiO_2$-Gel bei 100° C, und zwar jeweils unter Zugabe entsprechenden Sauerstoffs. $SO_2$ läßt sich mit Sauerstoff

zu $SO_3$ oxidieren, und zwar bei erhöhter Temperatur mit Hilfe eines Platin-Katalysators, mit Hilfe eines Katalysators aus $Fe_2O_3$ und mit $V_2O_5$ als Katalysator. CO läßt sich mit Hilfe von Palladium bei Temperaturen um oder höher als 150° C zu $CO_2$ oxidieren. Mittels eines Silber-Katalysators läßt sich bei 200 bis 400° C Methanol zu HCHO oxidieren.

Weitere katalytische Prozesse sind aus "Gmelins Handbuch der organischen Chemie", aus Winnacker-Küchler, "Chemische Technologie", aus Ullmans, "Enzyklopädie der technischen Chemie" und aus Reich, "Thermodynamik", bekannt.

Weitere Druckschriften, die Halbleitersensoren betreffen sind:
IEEE Trans. on Biomed. Eng., Vol. BME 19, (1972), S. 342-351,
IEEE Trans. on Biomed. Eng., Vol. BME 19, (1972), S.70-71,
Umschau, (1970), S. 651,
Umschau, (1969), S. 348,
DE-C-1 090 002
US-C-3 865 550.

Im Zusammenhang mit selektiver Wirkung für Gase sind Zeolithe bekannt, die auch als Molekularsieb bezeichnet werden. Solche Molekularsiebe haben die Eigenschaft Moleküle bestimmter Größenwerte und kleiner durchzulassen und größere Moleküle am Durchtritt zu hindern. Zahlreiche Beispiele verwendbarer Zeolithe sind bekannt aus:
Grubner u.a. "Molekularsiebe" VEB Dt. Verl. d. Wissensch., Berlin (1968).

Aufgabe der vorliegenden Erfindung ist es, eine Betriebsweise einen für Gasanalyse geeigneten Sensor anzugeben, so daß man in der Lage ist, ein vorgebbar einzelnes Gas, das ggf. eine Gaskomponente eines Gasgemisches ist, und insbesondere simultan selektiv mehrere einzelne, in einem Gasgemisch enthaltene Gaskomponenten zu detektieren bzw. das Vorhandensein eines nicht bekannten einzelnen Gases ggf. neben anderen Gasen festzustellen oder zu identifizieren.

Diese Aufgabe wird mit einem Betriebsverfahren mit den Merkmalen des Patentanspruchs 1 gelöst und aus den Unteransprüchen gehen weitere Ausgestaltungen und Weiter-bildungen der Erfindung hervor.

Die Erfindung beruht zum einen auf der Tatsache, daß Gassensoren, z.B. solche mit MOS-Halbleiterelementen, die katalytisch wirkendes Metall oder dgl. und/oder eine Zeolithschicht haben, für verschiedene Gase unterschiedlich große Anklingbzw. Ansprech- respektive Abkling- bzw. Abfall-Zeitkonstanten besitzen. Diese Zeitkonstante ist die Dauer, bis ein vorgebbar bestimmter Bruchteil des stationären Endwertes des Detektorsignals nach Einsetzen bzw. nach Beendigung des Einwirkens eines Gases erreicht ist. Bisher wurde derartiges zeitverzögertes Ansprechen eines Gassensors als Nachteil desselben angesehen und die Gasdetektion wurde z.B. erst nach Erreichen des stationären Zustandes durchgeführt.

Es ist festgestellt worden, daß der mit einem gegebenen Sensor bzw. dessen Detektor für ein bestimmtes Gas bzw. dessen Konzentration zu erhaltende Wert der Ansprech- (bzw. Abfall-) Zeitkonstanten abhängig von der (während der Messung vorliegenden) Betriebstemperatur des Detektors ist. Es ist auch festgestellt worden, daß unterschiedliche Gase bei ein und demselben Sensor bzw. Detektor voneinander verschiedene, jeweils für das einzelne Gas charakteristische Funktion einer solchen Temperaturabhängigkeit haben.

Eine noch weitergehende Feststellung ist, daß während der Meß-Zeitdauer bewirkte Änderung und insbesondere Wobbeln bzw. Modulation (periodisch auf und ab erfolgende Schwankungen) der Betriebstemperatur des betreffenden Sensors für jeweiliges Gas und dessen Konzentration charakteristische Änderung bzw. Schwankungen des Detektorsignals liefert. Es ist nämlich möglich, Gassensoren bzw. deren Detektoren so aufzubauen, daß deren Betriebstemperatur zeitlich sich rasch, z.B. im Bereich zwischen Sekunden und bis herab zu Millisekunden, verändern bzw. modulieren läßt, wohingegen die Meßdauer zur Ermittlung eines jeweiligen Meßwertes der Ansprech-(bzw. Abfall-)Zeitkonstanten Zeitdauern von Sekunden bis Minuten erfordern kann.

Im Rahmen der Erfindung liegt also die weitere Ausgestaltung, während der Meßwertermittlung die Betriebstemperatur eines Sensors (mit einer vorgegebenen Frequenz) zu modulieren und das als für ein Gas charakteristische Größe sich ergebende (mit gleicher Frequenz) schwankende Meßsignal festzustellen. Es ist festgestellt worden, daß außerdem Abhängigkeit von der gewählten Frequenz der Modulation vorliegt. Von besonderem Vorteil ist ggf., die jeweils auftretende Phasenverschiebung zwischen der schwanken-den Betriebstemperatur des Sensors und dem dementsprechend modulierten Detektorsignal als weiter auszuwertendes Signal festzustellen. Es wird sozusagen die Trägheit der Detektorinformation bezüglich der Temperaturänderungen ausgenutzt.

Eine weitere von der Modulationsfrequenz (der Änderungen der Betriebstemperatur) charakteristisch abhängige Information ist die sich jeweils ergebende Amplitude des zu erhaltenden Detektorsignals, nämlich für konstante Konzentration.

Wegen der für jeweiliges Gas charakteristischen Abhängigkeiten bzw. Funktion kann man durch Ausnutzung der oben erläuterten Möglichkeiten erreichen, daß man schon mit ein und demselben Sensor (und Änderungen seiner Betriebstemperatur) z.B. angeben kann, welches von beispielsweise vier in Frage kommenden Gasen (auf die voraussetzungsgemäß der Sensor ansprechen kann) im Augenblick der Messung auf den Sensor einwirkt bzw. vorliegt. Mit einer Mehrzahl von Sensoren, insbesondere solchen, die infolge unterschiedlichen Aufbaues (schon bei unveränderter Betriebstemperatur) voneinander verschiedene Gas-Sensitivität haben, lassen sich durch Variation, insbesondere (unterschiedliche) Modulation der Betriebstemperatur weitere charakteristische Sensitivitäten, d.h. weitere Selektivitäten hinsichtlich verschiedener Gase und/oder Gaskonzentrationen nutzbar machen.

Es kann die Ansprech-Zeitkonstante ermittelt und ausgewertet werden, und zwar mit bei der Durchführung der Messung relativ plötzlichem Einsetzen des Einwirkens des Gases bzw. der Gasmischung auf den Sensor bzw. auf dessen Detektor. Entsprechend ist sinngemäß relativ plötzlich endendes Einwirken für Messung der Abfall-Zeitkonstanten vorzusehen. "Relativ plötzlich" heißt dabei: in relativ zur Meßdauer kurzer Zeit von Millisekunden bis zu vielen Sekunden. Die als Detektorsignal gemessene Zeitkonstante soll nicht nennenswert durch langsames Anklingen bzw. langsamen Abfall des Kontaktes zwischen dem Gas (-Gemisch) und dem Detektor verfälscht sein.

Das Wiederabklingen (bei Messung der Ansprech-Zeitkonstanten) bzw. das Anklingen (bei Messung der Abfall-Zeitkonstanten) des Gases bzw. der Gasmischung, und zwar nach der Messung des Detektorsignals, spielt im Regelfall geringere Rolle. Zum Beispiel kann vorgesehen sein, daß der Sensor ständig dem Gas(-Gemisch) ausgesetzt sein soll, z.B. damit nicht außerdem noch Alterungseffekte eine Rolle spielen, so daß man dann die Abfall-Zeitkonstante als Meßwert nimmt. Entsprechend wird man bei zu detektierendem aggressivem Gas oder bei toxischen Gasen die Messung der Anstiegs-Zeitkonstanten bevorzugen und die Gaszufuhr möglichst bald wieder beenden.

Soweit hier von Gasen, Gaskomponenten und Gasgemischen die Rede ist, sind diesen sinngemäß Dämpfe und dgl. zuzurechnen.

Durch verschiedene Betriebstemperaturen lassen sich also mit der Erfindung jeweils mehrere voneinander unabhängige Meßergebnisse erzielen, deren Vergleich miteinander ein Auswertesignal mit klarer Aussage hinsichtlich Vorhandenseins einer Gaskomponente liefert. Soweit z.B. nicht zeitlich nacheinander die Messungen bei verschiedenen Betriebstemperaturen des Detektors des Gassensors durchgeführt werden, läßt sich mit Hilfe mehrerer gleichzeitig betriebener Einzeldetektoren, die auf untereinander verschiedene Betriebstemperaturen gebracht sind, eine Vielzahl voneinander unabhängiger Meßwerte erzielen.

Entsprechendes gilt für den Fall der Verwendung eines Detektors mit Zeolithschicht oder mehrerer Detektoren mit voneinander unterschiedlichen Zeolithschichten, wobei diese mehreren Detektoren vorzugsweise ebenfalls in einem Array angeordnet sind. Auch können in einem Array angeordnete Einzeldetektoren vorgesehen sein, die mit gleichen und/oder verschiedenen Zeolithen versehen sind und außerdem noch auf voneinander verschiedene Betriebstemperaturen zu bringen sind. Es kann damit eine Vielfalt von Unterscheidungskriterien der Einzeldetektoren genutzt werden.

Bei einem Gassensor ist bei der vorliegenden Erfindung vorgesehen, das Gas stoßweise fließen zu lassen, und zwar gesteuert durch beispielsweise Magnetventile, deren Öffnungs- bzw. Schließzeiten ausreichend klein im Verhältnis zu den Ansprech- (bzw. Abfall-)Zeitkonstanten sind. Diese Zeitkonstanten liegen im Bereich von Sekunden bis Minute, so daß genügend kurzzeitiges Öffnen (bzw. Schließen) durchführbar ist.

Bei den vorzugsweise verwendeten Halbleiter-Detektoren als dynamisch arbeitender Gassensor ist der Vorteil genutzt, daß ein Heizwiderstand zum Einstellen einer erhöhten Betriebstemperatur in integrierter Bauweise hinzugefügt werden kann. Ein solcher Heizwiderstand wird auf dem Siliziumchip angeordnet, auf dem sich auch das Halbleiter-Detektorelement des Sensors befindet.

Besonders vorteilhaft ist es, die Modulationsfrequenz so zu bemessen, daß sie etwa gleich dem Kehrwert der Ansprech-Zeitkonstanten für die Hauptempfindlichkeit eines jeweils bestimmten Gases, bzw. einer bestimmten Gaskomponente ist. Für einen Einzeldetektor ergeben sich für verschiedene Gase, bzw. Gaskomponenten unterschiedliche Zeitkonstanten. Durch Wahl verschiedener Modulationsfrequenzen bei diesem einen Einzeldetektor kann man somit eine Hauptempfindlichkeit für ein jeweiliges Gas erzeugen. Damit kann man einen einzigen Einzeldetektor durch Wahl der Modulationsfrequenz für verschiedene Gase bevorzugt selektiv machen.

Gemäß einer Weiterbildung der Erfindung ist vorgesehen, ein Array aus mehreren Einzeldetektoren, insbesondere aus gleichaufgebauten Einzeldetektoren zu benutzen. Um mit diesem Array mehrere Gaskomponenten, insbesondere simultan, voneinander unterscheiden zu können, werden gemäß dieser Ausgestaltung dieser Erfindung Einzeldetektoren dieses Arrays mit voneinander verschiedenen Modulationsfrequenzen der Temperaturmodulation des betreffenden Einzeldetektors betrieben. Aus der US-PS 4 457 161 ist ein aus mehreren Einzeldetektoren bestehendes Array bekannt, die in Form einer Matrix mit i Zeilen und j Spalten (ixj = n) angeordnet sind. Mit diesem Array werden Einzelkomponenten eines (Gas-)Gemisches voneinander unterschieden und detektiert. Die Auswertung beruht auf der mathematischen Lösung eines linearen Gleichungssystems aus n Gleichungen. Jede einzelne Gleichung repräsentiert das Empfindlichkeits-Spektrum der Summe der Einzeldetektoren einer Zeile bzw. einer Spalte oder eines jeden Einzeldetektors hinsichtlich jeder der Einzelkomponenten des Gemisches, wobei eine einzelne Einzelempfindlichkeit auch gleich Null sein kann. Unterschiedliches Empfindlichkeits-Spektrum der Einzeldetektoren bzw. Detektorzeilen und -spalten kann auf unterschiedlichen Temperaturen beruhen.

Der Vollständigkeit halber sei darauf hingewiesen, daß die thermische Zeitkonstante des Einzeldetektors kürzer als die Ansprechzeitkonstante für die gegebene Gaskomponente sein muß, um eine wirkungsvolle Temperaturmodulation zu erzielen.

Die Modulation der Temperatur kann entweder durch eigens dazu angebrachte Heiz- oder Kühlelemente erfolgen oder durch eine Modulation des Arbeitspunktes und damit eine Modulation der Verlustleistung. Dies kann durch den eingeprägten Diodenstrom, bei einem MOS-Transistor durch Modulation der Substratspannung erzielt werden.

Bei dieser Weiterbildung können die Vorteile der Wechselstrommeßtechnik hinsichtlich der Verbesserung des Signal-Geräuschverhältnisses durch Bandbreitebegrenzung genutzt werden.

Vorzugsweise wird ein erfindungsgemäßer bei der Erfindung verwendeter Detektor durch Reihenversuche geeicht.

Weitere Erläuterungen der Erfindung gehen aus der nachfolgenden, anhand der Figuren gegebenen Einzelbeschreibung hervor.

Figuren 1 und 2 zeigen ein Ausführungsbeispiel in seitlicher Schnittansicht (Fig. 1) und in Aufsicht (Fig. 2).

Figur 3 zeigt eine Meßanordnung und
Figuren 4 und 5 zeigen ein Detektorarray und dazu das Prinzip einer Mustererkennung.

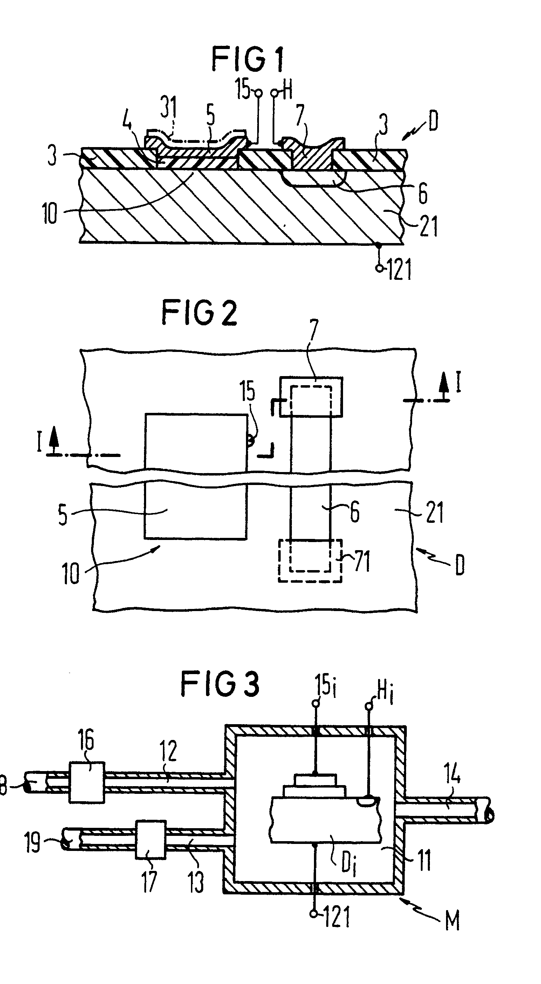

Figur 1 zeigt einen Sensor D mit einem MOS-Detektor 10 mit integriertem Heizwiderstand 6 zur wahlweisen Einstellung einer jeweils vorgebbaren Betriebstemperatur. Auf dem Halbleiter-Substratkörper 21 aus z.B. p-leitendem Silizium befindet sich eine Schutzschicht 3 aus Siliziumdioxid. Mit 4 ist eine Gateoxid-Schicht aus vorzugsweise thermisch erzeugtem Siliziumdioxid bezeichnet. Die Metallschicht 5 aus beispielsweise Palladium ist die katalytisch wirksame Schicht. Sie hat den Anschluß 15.

Möglichst nahe diesem Detektor 10 mit Gateoxid-Schicht 4 und katalytischer Schicht 5 ist im Substratkörper 21 ein Bereich 6 vorgesehen, der z.B. entartet n-dotiert ist. Am einen Ende dieses Bereiches 6 ist auf der Oberfläche des Substratkörpers 21 eine Aluminium-Elektrode 7 mit ihrem Anschluß H angeordnet. Diese Anordnung ist ein Heizwiderstand. Dessen zweite Elektrode kann der Substratkörper 21 sein.

Weitere Aufschlüsse zu dieser Ausführungsform gibt die in Figur 2 gegebene Aufsicht auf den Sensor D nach Figur 1. Mit 21 sind der Substratkörper, mit 121 dessen Anschluß und mit 5 die katalytisch wirksame Metallschicht bezeichnet. Mit 71 ist alternativ eine zweite Elektrode für die als Heizer dienende Widerstandsbahn des Bereiches 6 angedeutet. Insbesondere kann dieser Bereich 6 um den Detektor 10, d.h. um die katalytische Schicht 5 herumgelegt, diesen möglichst weitgehend umringend, angeordnet sein, so daß der Detektor 10 von mehr als nur einer Seite auf seine vorgegebene Betriebstemperatur aufzuheizen ist.

In Figur 1 ist mit 31 eine fakultativ vorgesehene Zeolithschicht, z.B. ausgewählt aus an sich bekannten Zeolithen, bezeichnet. Bei einem Detektor mit Zeolithschicht 31 können somit für die Detektion verschiedener Gase dessen für verschiedene Gase unterschiedliche Zeitkonstanten genutzt werden. Dabei ergeben sich unterschiedliche Zeitkonstanten nicht nur für wahlweise vorgebbare unterschiedlich gewählte Betriebstemperaturen und/oder Modulationsfrequenz, sondern "zweidimensional" auch aufgrund der jeweils gewählten Zeolithen der Schicht 31.

Figur 3 zeigt eine Anordnung, die vorzugsweise zur Eichung, jedoch auch zum erfindungsgemäßen Betrieb geeignet ist. In einem mit 11 angedeuteten Volumen einer Meßkammer M ist der Sensor Di angeordnet. Dieses Volumen 11 hat zwei Einlässe 12 und 13 und einen Auslaß 14. Mit 16 und 17 sind je ein Magnetventil zur Steuerung von Gas-Stromstößen bezeichnet. Über die Leitung 18 wird das zu detektierende Gas zugeführt. Die Leitung 19 ist für die Zuführung eines Referenzgases vorgesehen. Der i-te Detektor Di hat den Anschluß 15i.

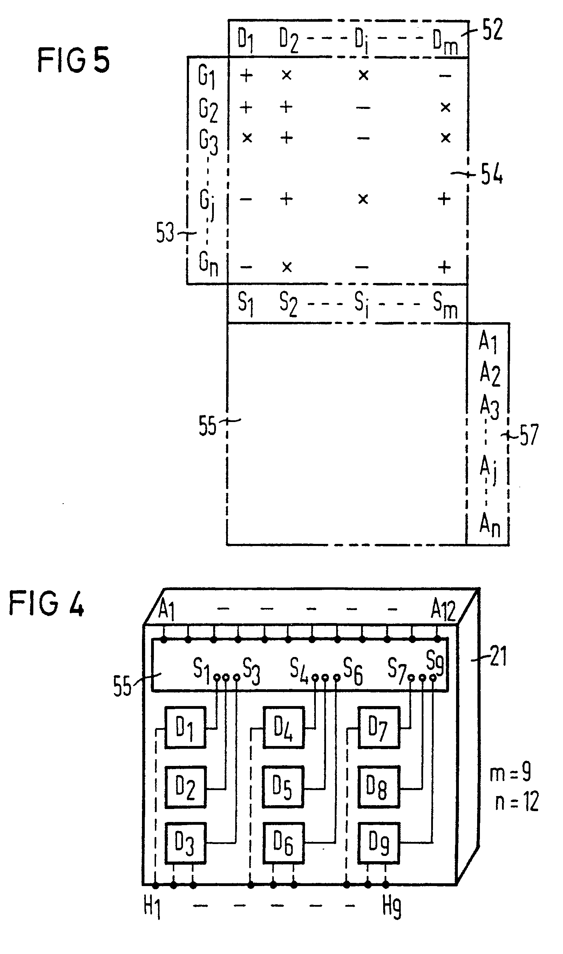

Figur 4 zeigt eine schematische Darstellung eines Detektorarrays mit neun Einzeldetektoren D1 bis D9 und einer Auswertung 55. Das Array hat vorzugsweise integrierten Aufbau auf einem Substratkörper 21 aus vorzugsweise Halbleitermaterial.

Es ist vorgesehen, daß die Detektoren D1 bis D9 einzeln auf voneinander verschiedenen Temperaturen bzw. ein oder mehrere dieser Detektoren auf von den übrigen (bei "Zimmertemperatur" betriebenen) Detektoren abweichender Temperatur gebracht oder gehalten werden. Hierzu dienen die angedeuteten Stromzuführungen $H_1$ bis $H_9$. Der Heizstrom kann auch moduliert sein, was zu entsprechend charakteristischer Empfindlichkeit führt, wie oben dargelegt. Mit unterschiedlicher selektiver Modulationsfrequenz kann unterschiedliche charakteristische Empfindlichkeit erreicht werden.

Figur 5 zeigt das Schema einer Mustererkennung, mit deren Hilfe eine (auch simultane) Auswertung der durch die Zeitkonstanten-Werte der einzelnen Sensoren $D_1$ bis $D_m$ gegebenen Ausgangssignale S1 bis Sm durchführbar ist.

Die schematische Darstellung der Figur 4 zeigt das Prinzip eines Gassensors mit einem Detektorarray mit den Einzeldetektoren D1, D2 bis Dm. Für die einzelnen Gase bzw. für die Gaskomponenten G1, G2, G3 ... Gi ... Gn eines Gasgemisches haben die Einzeldetektoren D1, D2... die in der Matrix 54 in der jeweiligen zugehörigen Spalte der Matrix 54 angegebenen Empfindlichkeiten. Ein Pulszeichen bedeutet hohe Empfindlichkeit bzw. Hauptempfindlichkeit, ein Andreaskreuz bedeutet dagegen deutlich mindere Empfindlichkeit und ein Minuszeichen steht für Unempfindlichkeit des betreffenden Einzeldetektors gegenüber der betreffenden Gaskomponente G1, G2 .... Die Einzeldetektoren bilden die Zeile 52 und die Gaskomponenten die Spalte 53 zur Matrix 54. Es sei darauf hingewiesen, daß eine solche Matrix z.B. auch lediglich nur zwei Einzeldetektoren D1 und D2 besitzt.

Die untere Zeile enthält die Signalausgänge 15i (i = 1,2,...) der Einzeldetektoren D1, D2 ..., die jeweilige Signale S1, S2 bis Sm liefern. Das Signal S1 z.B. ist der ermittelte Wert der Zeitkonstanten (wie oben dargelegt) des Einzeldetektors D1 gegenüber den Gaskomponenten G1, G2 bis Gn. Es enthält auch die Information, daß der Einzeldetektor D1 gegenüber den Gaskomponenten Gi und Gn unempfindlich ist. Entsprechende Zeitkonstantenwerte ergeben die übrigen Signale S2 bis Sm. Sofern z.B. die Gaskomponenten G2 und Gn nicht vorhanden sind, unterscheidet sich ein dann zu erhaltendes Signal S'1 vom Signal S1 darin, daß der ansonsten auf der Gaskomponente G2 beruhende Signalanteil, hier sogar eine Hauptempfindlichkeit des Einzeldetektors gegenüber der Gaskomponente G2, im Signal S'1 fehlt. Das Fehlen der Gaskomponente Gn liefert ersichtlich keinen Beitrag zum vorliegenden Unterschied von S'1 gegenüber S1. Das bei z.B. Fehlen der Gaskomponenten G2 und Gn auftretende Signal S'm unterscheidet sich vom Signal Sm darin, daß der Signalanteil der Hauptempfindlichkeit gegenüber der Gaskomponente Gn und die mindere Empfindlichkeit gegenüber der Gaskomponente G2, d.h. die durch diese Gase gegebenen Zeitkonstanten-Anteile fehlen.

Mit 55 ist eine Mustererkennungs-Matrix bezeichnet, die nach Art einer Logik arbeitet. Dieser Matrix werden wie ersichtlich die Detektorsignale, d.h. im jeweiligen Einzelfall die für eine Gaskomponentenmischung x tatsächlich auftretenden Signale S1 bis Sm zugeführt. Diese Matrix 55 ist in der Lage, aus der Gesamtheit der zugeführten Signale S1 bis Sm, d.h. aus der Anzahl m Signale auf das Vorhandensein bzw. Nichtvorhandensein einzelner Gaskomponenten aus einer in die Mustererkennungsmatrix einprogrammierten Anzahl n Gaskomponenten zu schließen. Dabei kann die Anzahl m sogar (um eine sprechende relative Zahl) kleiner als die Anzahl n sein. Es sei angemerkt, daß auch das Vorhandensein eines nicht-einprogrammierten Gases (aufgrund eines nicht zuzuordnenden Anteils in der gemessenen Zeitkonstanten) wenigstens festzustellen ist.

Der Matrix 54 entspricht mathematisch ausgedrückt das Gleichungssystem

$$Si = \sum_{j=1}^{j=n} (a_{ij}\, G_j)$$

mit i von 1 bis m für die Signale S1 bis Sm.

Die $a_{ij}$ mit j verschieden von i sind die oben erwähnten Querempfindlichkeiten.

Im Stand der Technik wurde und wird angestrebt, solche Detektoren zu entwickeln, die möglichst kleine Querempfindlichkeiten aufweisen, d.h. bei denen die Matrixelemente $a_{ij}$ für i verschieden von j möglichist klein gegenüber den Matrixelementen $a_{ij}$ mit i gleich j sind. Dies erfordert für jede Gaskomponente mindest einen eigenen Einzeldetektor, d.h. m muß gleich oder größer als n sein.

Bei der Erfindung dagegen werden die Querempfindlichkeiten $a_{ij}$ mit i verschieden von j in erfindungswesentlichem Maße genutzt und ausgewertet. Bei der Erfindung sind Querempfindlichkeiten gerade erwünscht, was dem bisherigen Entwicklungsstand sogar entgegengesetzt gerichtet ist. In der Ausnutzung der Querempfindlichkeiten ist begründet, daß bei der Erfindung die Anzahl m der Einzeldetektor ohne weiteres kleiner sein kann als die Anzahl n der zu detektierenden Gaskomponenten.

Wenn die $a_{ij}$ konstante Werte der jeweiligen Empfindlichkeit des betreffenden Einzeldetektors Si sind, eingeschlossen der Wert Null, ergibt sich ein lineares Gleichungssystem, das mit Hilfe der Hustererkennungsmatrix 55 gelöst wird. Sofern die $a_{ij}$ eine Funktion abhängig vom Vorhandensein der über die Gaskomponente Gj hinaus vorhandenen weiteren Gase G... ist, wird mit Hilfe entsprechender Eichung die Mustererkennungsmatrix 55 in die Lage versetzt, auch dieses Gleichungssystem zu lösen.

Es sind hierzu die entsprechenden Eichungen des aus den Einzeldetektoren bestehenden Detektorarrays unter Verwendung jeweils bekannter, unterschiedlicher Gasmischungen vorzunehmen. Entsprechendes gilt, wenn die Empfindlichkeiten $a_{ij}$ eine Funktion der vorliegenden Konzentration des jeweiligen Gases Gj für j = i und/oder der weiteren vorhandenen Gase Gj für j ≠ i ist. Die Mustererkennungsmatrix 55 wird dann derart ausgerüstet, daß sie Iterationen durchzuführen vermag, mit deren Hilfe auch in diesem Falle die eindeutige Zuordnung möglich ist, d.h. die Lösung auf an sich bekanntem mathematischem Wege mit Hilfe der Mustererkennungsmatrix 5 zu erhalten ist.

Das Verfahren der Eichung und Mustererkennung kann mathematisch auch als eine Art der Bildung von Korrelationskoeffizienten verstanden werden. Dazu folgendes Beispiel: Für jede zum Zwecke der Eichung vorgegebene Gaskomponente Gj werden in dem Eichverfahren die Signale $S^*_{ij}$ für j von 1 bis n vermittelt. Diese i . j Werte von $S^*_{ij}$ werden in einem Speicher der Mustererkennungsmatrix 5 abgespeichert. Bei der Messung des zu bestimmenden Gasgemisches werden die Korrelationskoeffizienten $ß_j$ gemäß folgender Vorschrift bestimmt:

$$ß_j = \underset{i = 1}{\overset{i = m}{Summe}} \ S_i \ S^*_{ij}$$

Der Korrelationskoeffizient $ß_j$ gibt dann den Anteil der zu bestimmenden Gaskomponenten $G_j$ an.

Die Mustererkennungs-Matrix 55 hat die in Spalte 57 angegebenen Ausgänge A1 bis An für die Anzahl n Gaskomponenten G1 bis Gn. An diesen Ausgängen A lassen sich die Einzelwerte für die betreffenden Gaskomponenten abnehmen.

**Patentansprüche**

1. Verfahren zum Betrieb eines Sensors zur Gasanalyse, der mit einem elektrischen Signal auf das Vorhandensein eines oder mehrerer Gase oder Gaskomponenten eines Gasgemisches anspricht, wobei dieses Signal nach Ablauf einer Zeitdauer seinen Endwert erreicht und der dynamische Verlauf des Signalwertes gemessen wird,
   **gekennzeichnet** dadurch,
   daß der Sensor (D) bwz. sein Detektor (10) für eine jeweilige Messung momentan einsetzend bzw. momentan endend mit dem Gas bzw. dem Gasgemisch beaufschlagt wird und daß während der Meßdauer die Ansprech- bzw. Abfall-Zeitkonstante des Sensors (D) bzw. Detektors (10) als Detektorsignal (S) festgestellt und als Ausgangssignal (A) ausgewertet wird.

2. Verfahren nach Anspruch 1, **gekennzeichnet** dadurch, daß der Detektor (10) im Sensor (D) wenigstens während der Meßdauer auf einer von der Umgebungstemperatur abweichenden Betriebstemperatur gehalten wird.

3. Verfahren nach Anspruch 1, **gekennzeichnet** dadurch, daß die Ermittlung des Detektorsignals (S) mit einem Sensor (D) durchgeführt wird, dessen Detektor (10) hinsichtlich seiner Temperaturänderungen eine Zeitkonstante hat, die kleiner als diejenige Meßdauer ist, die für diese Ermittlung angesetzt wird.

4. Verfahren nach Anspruch 1, 2 oder 3, **gekennzeichnet** dadurch, daß der Detektor (10) im Sensor (D) während der Meßdauer von einer vorgegebenen Temperatur auf eine andere vorgegebene Temperatur gebracht wird.

5. Verfahren nach Anspruch 1, 2 oder 3, **gekennzeichnet** dadurch, daß die Betriebstemperatur des Detektors (10) im Sensor (D) während der Meßdauer moduliert wird.

6. Verfahren nach Anspruch 5, **gekennzeichnet** dadurch, daß diese Modulation mit vorgegebener Modulationsfrequenz erfolgt.

7. Verfahren nach Anspruch 5 oder 6, **gekennzeichnet** dadurch, daß als Meßwert die Phasenverschiebung zwischen der Modulation der Temperatur des Detektors (10) und dem diese Modulationsfrequenz enthaltenden Detektorsignal (S) ausgewertet wird.

8. Verfahren nach einem der Ansprüche 1 bis 7, **gekennzeichnet** dadurch, daß diese Ermittlung des Detektorsignals (S) mit einem Sensor (D) einem Detektor (10) mit einer katalytisch wirkenden Schicht (5) durchgeführt wird.

9. Verfahren nach einem der Ansprüche 1 bis 8, **gekennzeichnet** dadurch, daß die Ermittlung des Detektorsignals (S) mit einem Sensor (D) mit einem Detektor (10) durchgeführt wird, der eine Zeolithschicht (31) besitzt.

10. Verfahren nach einem der Ansprüche 1 bis 9, **gekennzeichnet** dadurch, daß die Analyse unter Verwendung einer Anzahl Sensoren (D1 ... Dm) durchgeführt wird, die gegenüber einem Gas oder Gasgemisch selektiv voneinander unterschiedliche charakteristische Ansprech-Zeitkonstanten aufweisen.

11. Verfahren nach Anspruch 10, **gekennzeichnet** dadurch, daß diese Anzahl Sensoren (D1 ... Dm) als Array nach Art einer Matrix (Fig. 4) zusammengefaßt sind und
    die Auswertung (57) der Anzahl m der gemessenen Detektorsignale (S1 ... Sm) nach dem Prinzip einer Mustererkennung (55; Fig. 5) durchgeführt wird.

12. Verfahren nach Anspruch 11, **gekennzeichnet** dadurch, daß ein Array verwendet wird, das wenigstens mehrere Sensoren (Di) hat, die auf während der Messung der Detektorsignale untereinander verschiedene Temperatur gebracht werden oder auf verschiedener Temperatur gehalten werden.

13. Verfahren nach Anspruch 12, **gekennzeichnet** dadurch, daß für diese Sensoren (Di) eine Schicht (31) aus ein und demselben Zeolithmaterial verwendet wird.

14. Verfahren nach Anspruch 11 oder 12, **gekennzeichnet** dadurch, daß ein Array verwendet wird, das wenigstens mehrere Sensoren (Di) hat, die eine Schicht (31) aus jeweils voneinander verschiedenem Zeolithmaterial haben.

15. Verfahren nach einem der Ansprüche 11 bis 14, **gekennzeichnet** dadurch, daß mit dem Array mit den Sensoren (D1, D2 ...Dm) eine Anzahl n einzelner Gase bzw. Gaskomponenten detektiert werden, wobei diese Anzahl n kleiner als die Anzahl m der Sensoren (D1 ...Dm) sein kann.

## Claims

1. Method of operating a sensor for gas analysis, which responds with an electrical signal to the presence of one or more gases or gas components of a gas mixture, this signal achieving its final value upon expiry of a time interval, and the dynamic response of the signal value being measured, characterised in that for a particular measurement the sensor (D) or its detector (10) is influenced in an instantaneously starting or instantaneously terminating fashion by the gas or the gas mixture, and in that during the measurement period the response time constant or decay time constant of the sensor (D) or detector (10) is determined as a detector signal (S) and evaluated as an output signal (A).

2. Method according to Claim 1, characterised in that the detector (10) in the sensor (D) is maintained at least during the measurement period at an operating temperature different from the ambient temperature.

3. Method according to Claim 1, characterised in that the determination of the detector signal (S) is carried out by means of a sensor (D), whose detector (10) has a time constant with respect to its temperature variations which is smaller than that measurement period which is set for this determination.

4. Method according to Claim 1, 2 or 3, characterised in that during the measurement period the detector (10) in the sensor (D) is brought from one prescribed temperature to another prescribed temperature.

5. Method according to Claim 1, 2 or 3, characterised in that the operating temperature of the detector (10) in the sensor (D) is modulated during the measurement period.

6. Method according to Claim 5, characterised in that this modulation is performed using a prescribed modulation frequency.

7. Method according to Claim 5 or 6, characterised in that the phase shift between the modulation of the temperature of the detector (10) and the detector signal (S) containing this modulation frequency is evaluated as the measured value.

8. Method according to one of Claims 1 to 7, characterised in that this determination of the detector signal (S) is carried out by means of a sensor (D) with a detector (10) having a catalytically active layer (5).

9. Method according to one of Claims 1 to 8, characterised in that the determination of the detector signal (S) is carried out by means of a sensor (D) with a detector (10) which possesses a zeolite layer (31).

10. Method according to one of Claims 1 to 9, characterised in that the analysis is carried out using a number of sensors (D1...Dm), which have characteristic response time constants differing selectively from one another with respect to a gas or gas mixture.

11. Method according to Claim 10, characterised in that this number of sensors (D1...Dm) are combined as a matrix-type array (Fig. 4), and the evaluation (57) of the number m of the measured detector signals (S1...Sm) is carried out according to the principle of pattern recognition (55; Fig. 5).

12. Method according to Claim 11, characterised in that use is made of an array which has at least a plurality of sensors (Di), which are brought to temperatures differing from one another during the measurement of the detector signals, or are maintained at different temperatures.

13. Method according to Claim 12, characterised in that a layer (31) of one and the same zeolite material is used for these sensors (Di).

14. Method according to Claim 11 or 12, characterised in that an array is used which has at least a plurality of sensors (Di), which have a layer (31) of zeolite material differing respectively from one another.

15. Method according to one of Claims 11 to 14, characterised in that a number n of individual gases or gas components can be detected by means of the array with the sensors (D1, D2...Dm), it being possible for this number n to be smaller than the number m of the sensors (D1...Dm).

**Revendications**

1. Procédé d'utilisation d'un capteur pour analyser des gaz, qui réagit par un signal électrique à la présence d'un gaz ou de plusieurs gaz ou de constituants gazeux d'un mélange de gaz, ce signal atteignant sa valeur définitive après l'écoulement d'un certain laps de temps et la variation dynamique de la valeur du signal étant mesurée,

   caractérisé en ce qu'il consiste à alimenter en le gaz ou en le mélange de gaz le capteur (D) ou son détecteur (10) pour une mesure débutant instantanément et se terminant instantanément et à déterminer, pendant la durée de la mesure, la constante de temps de réponse ou de décroissance du capteur (D) ou du détecteur (10) en tant que signal du détecteur (S) et à l'exploiter comme signal de sortie.

2. Procédé suivant la revendication 1, caractérisé en ce qu'il consiste à maintenir le détecteur (10) du capteur (D), au moins pendant la durée de la mesure, à une température de fonctionnement différente de la température ambiante.

3. Procédé suivant la revendication 1, caractérisé en ce qu'il consiste à effectuer la détermination du signal du détecteur (S) avec un capteur (D) dont le détecteur (10) a, du point de vue de ses variations de température, une constante de temps qui est inférieure à la durée de mesure qui est fixée pour cette détermination.

4. Procédé suivant la revendication 1, 2 ou 3, caractérisé en ce qu'il consiste à porter le détecteur (10) du capteur (D), pendant la durée de la mesure, d'une température prescrite à une autre température prescrite.

5. Procédé suivant la revendication 1, 2 ou 3, caractérisé en ce qu'il consiste à moduler la température de fonctionnement du détecteur (10) du capteur (D) pendant la durée de la mesure.

6. Procédé suivant la revendication 5, caractérisé en ce qu'il consiste à effectuer cette modulation avec une fréquence de modulation prescrite.

7. Procédé suivant la revendication 5 ou 6, caractérisé en ce qu'il consiste à exploiter, comme valeur de mesure, le déphasage entre la modulation de la température du détecteur (10) et le signal du détecteur (S) contenant cette fréquence de modulation.

8. Procédé suivant l'une des revendications 1 à 7, caractérisé en ce qu'il consiste à effectuer cette détermination du signal du détecteur (S) par un capteur (D) ayant un détecteur (10) à couche (5) à action catalytique.

9. Procédé suivant l'une des revendications 1 à 8, caractérisé en ce qu'il consiste à effectuer la détermination du signal du détecteur (S) par un capteur (D) ayant un détecteur (10) qui possède une couche de zéolite (31).

10. Procédé suivant l'une des revendications 1 à 9, caractérisé en ce qu'il consiste à effectuer l'analyse en utilisant un nombre de capteurs (D1 ... Dm) qui présentent des constantes de temps de réponse caractéristiques différentes l'une de l'autre, sélectivement pour un gaz ou pour un mélange de gaz.

11. Procédé suivant la revendication 10, caractérisé en ce que ce nombre de détecteurs (D1 ... Dm) est rassemblé sous la forme d'un réseau, à la manière d'une matrice (figure 4), et

   l'exploitation (57) du nombre m des signaux de détecteurs (S1 ... Sm) mesurés est effectuée par le principe de la reconnaissance de structures (55 ; figure 5).

12. Procédé suivant la revendication 11, caractérisé en ce qu'il consiste à utiliser un réseau qui a au moins plusieurs capteurs (Di) qui sont portés, pendant la mesure des signaux de détecteur, à des températures différentes entre elles ou qui sont maintenus à des températures différentes.

13. Procédé suivant la revendication 12, caractérisé en ce qu'il consiste à utiliser, pour ces capteurs (Di), une couche (31) en une seule et même matière zéolite.

**14.** Procédé suivant la revendication 11 ou 12, caractérisé en ce qu'il consiste à utiliser un réseau ayant au moins plusieurs capteurs (Di) qui ont chacun une couche (31) en une matière zéolite différente.

**15.** Procédé suivant l'une des revendications 11 à 14, caractérisé en ce qu'il consiste à détecter, avec le réseau ayant les capteurs (D1, D2 ... Dm), un nombre n de gaz individuels ou de constituants gazeux, ce nombre n pouvant être inférieur au nombre m des capteurs (D1 ... Dm).

## FIG 1

## FIG 2

## FIG 3

FIG 5

| | D$_1$ | D$_2$ | - - - | D$_i$ | - - - | D$_m$ |
|----|----|----|----|----|----|----|
| G$_1$ | + | × | | × | | − |
| G$_2$ | + | + | | − | | × |
| G$_3$ | × | + | | − | | × |
| G$_j$ | − | + | | × | | + |
| G$_n$ | − | × | | − | | + |

52

53

54

S$_1$  S$_2$ - - - S$_i$ - - - S$_m$

A$_1$
A$_2$
A$_3$
A$_j$
A$_n$

55

57

FIG 4

A$_1$  −  −  −  −  −  A$_{12}$

21

55

S$_1$  S$_3$     S$_4$  S$_6$     S$_7$  S$_9$

D$_1$     D$_4$     D$_7$

D$_2$     D$_5$     D$_8$

D$_3$     D$_6$     D$_9$

m = 9
n = 12

H$_1$  −  −  −  −  −  H$_9$

12